(19) Europäisches Patentamt
European Patent Office
Office européen des brevets

(11) **EP 2 336 304 A1**

(12) **EUROPEAN PATENT APPLICATION**

(43) Date of publication:
**22.06.2011 Bulletin 2011/25**

(51) Int Cl.:
**C12N 7/02** *(2006.01)*    **B01D 15/08** *(2006.01)*

(21) Application number: **10194894.1**

(22) Date of filing: **14.12.2010**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **16.12.2009 US 284368 P**

(71) Applicant: **Millipore Corporation**
**Billerica, MA 01821 (US)**

(72) Inventors:
  • **Iyer, Ganspathysubramanian**
    **Billerica, MA 01821 (US)**

  • **Ramaswarmy, Senthil**
    **Billerica, MA 01821 (US)**
  • **Cheng, Kwok-Shun**
    **Billerica, MA 01821 (US)**

(74) Representative: **Gambell, Derek et al**
**Graham Watt & Co LLP**
**St Botolph's House**
**7-9 St Botolph's Road**
**Sevenoaks**
**Kent TN13 3AJ (GB)**

(54)    **Flow through purification processes for large biomolecules**

(57)    The present invention relates, at least in part, to novel and improved flow-through purification processes for separating large biomolecules, such as, for example, encapsulated viruses, virus-like particles and conjugate vaccines from one or more contaminants in a sample, where the process employs the use of at least one population of a solid porous particle which comprises a minimized external surface area per unit volume of the particles and an internal surface area per unit volume which is not decreased by more than 25% relative to a population of a similar particle which does not have a minimized external surface area.

Fig.1

EP 2 336 304 A1

**Description**

**Related Applications**

The present application claims the benefit of priority of U.S. Provisional Patent Application No. 61/284,368, having the filing date of December 16, 2009, the entire content of which is incorporated by reference herein in its entirety.

**Field of Invention**

The present invention relates, at least in part, to novel and improved flow-through purification processes for separating large biomolecules, such as, for example, encapsulated viruses, virus-like particles and conjugate vaccines from one or more contaminants in a sample.

**Background of the Invention**

Following the production of a large biomolecule such as, for example, an encapsulated virus, a virus-like particle in either infected host cells or eggs or a conjugate vaccine, it is desirable to separate the biomolecule from other components of the infected host cells or eggs, such as, for example, DNA, RNA and host cell proteins, in order to obtain a substantially pure population of the biomolecule. Further, the biomolecule being produced needs to be separated from several additives which are typically used for facilitating the production of the biomolecule, as the additives are often capable of being co-purified with the biomolecule.

Conventionally, a variety of techniques such as ultra-centrifugation, chromatography and membrane filtration have been used to purify and concentrate large biomolecules such as encapsulated viruses and virus-like particles (see, e.g., PCT Publication Nos. W02008/073490 and WO2004/112707) The various types of chromatography techniques which have been used to purify such biomolecules include, for example, size exclusion (SEC), anion exchange (AEX) and affinity chromatography (see, *e.g.,* WO2004/112707 Transfiguracuin et al., J Virol Methods 142:21-28 (2007) and Kalbfuss et al. Biotech. Bioeng. 96: 932-944 (2007)).

While the foregoing techniques are being used to some extent for the purification of large molecules such as encapsulated viruses and virus-like particles, all of these chromatographic techniques are generally used in the bind and elute mode. Specifically, due to the large size of such biomolecules, (e.g., ~15 -400 nM), they are unable to penetrate the pores of most commercially available media, and therefore, they typically bind to the outside of the media and are subsequently eluted. Whereas, the contaminants and impurities generally bind to the internal surface area of the particles, however, sometimes partially elute with the large biomolecule. Further, sometimes, one or more of these techniques are used in a non-continuous mode, however, generally the overall yields are poor.

**Summary of the Invention**

This present invention provides, at least in part, an improved flow-through process for the separation of large biomolecules such as, for example, encapsulated viruses, virus-like particles and conjugate vaccines from one or more contaminants in a sample. The present invention employs one or more chromatography techniques including, for example, anion exchange chromatography (AEX), cation exchange chromatography (CEX) and hydrophobic interaction chromatography (HIC) to purify large biomolecules such as encapsulated viruses, virus-like particles, conjugate vaccines and bacterial cell surface polysaccharides, in a flow-through mode.

The methods according to the present invention typically result in an improved recovery of a large biomolecule such as a virus, a virus-like particle or a conjugate vaccine using a single continuous flow-through process, which is far superior to the conventional methods currently used in the art, which are generally a bind and elute mode and are non-continuous. The methods according to the present invention are based, at least in part, on the use of at least one type of chromatography particle (e.g., a solid porous bead), for which the external surface area per unit volume is minimized and the internal surface area per unit volume is not decreased by more than 25%, relative to a similar chromatography particle which is typically used in a chromatography process, however, for which the external surface area per unit volume is not minimized. In general, the present invention relies, at least in part, on a reverse mode of operation, *i.e.,* by using one or more of AEX, CEX and HIC chromatography media in a flow-through mode, where at least one of such media comprises the characteristics which result in an improved recovery, e.g., a minimized external surface area per unit volume and an internal surface area per unit volume, which is not decreased by more than 25%.

The methods according to the present invention allow for recovery of a large biomolecule in a single step process as well as provide for minimal upstream and down stream processing of the feed and product stream as compared to conventional chromatographic methods which require a number of pre- and post- purification steps. Further, the methods of the present invention result in improved recovery of a large biomolecule both in the presence and in the absence of host cell proteins in a sample containing the large biomolecule.

In one aspect of the present invention, a flow-through process for improving recovery of a large biomolecule from a sample comprising the large biomolecule and one or more contaminants is provided. Such a process comprises contacting the sample with one or more populations of solid porous particles having a molecular weight cut off smaller than the large biomolecule being recovered, where the external surface area per unit volume of at least one population of a solid porous particle used in the flow-through process is minimized and where the internal surface area per unit volume is not decreased by more than 25%, relative to a population of the solid porous particle which does not comprise a minimized external surface area per unit volume, thereby to improve recovery of the large biomolecule.

In some embodiments, the one or more populations of solid porous particles are packed in a chromatography column.

The flow-through processes according to the present invention result in an improved recovery of a large biomolecule, for example, by at least 10%, or at least 20%, or at least 30%, or at least 40%, or at least 50%, or at least 60%, or at least 70%, or at least 80%, or at least 90%, over recovery of the large biomolecule using a flow-through process in which at least one population of a solid porous particle comprising a minimized external surface area per unit volume and an internal surface area per unit volume which is not decreased by more than 25%, is not used.

In some embodiments, a flow-through process according to the present invention results in an improved purity of the large biomolecule by at least 20%.

In some embodiments, a flow-through process according to the present invention results in a decrease in one or more contaminants by at least 10%, or at least 20%, or least 30%, or at least 40% or more.

In some embodiments, the one or more populations of solid porous particles are selected from one or more of beads used for anion exchange chromatography, cation exchange chromatography and hydrophobic interaction chromatography.

A large biomolecule which can be recovered using the processes of the present invention is selected from the group consisting of an encapsulated virus, a virus-like particle and a conjugate vaccine.

In some embodiments, the one or more contaminants is selected from the group consisting of nucleic acid, proteins, excipients and cell culture additives. In some embodiments, the one or more contaminants are host cell proteins.

In some embodiments, a flow-through process according to the invention is used for separating a virus-like particle from one or more contaminants. An exemplary virus-like particle includes, but is not limited to, a styrene particle coated with BSA.

In some embodiments, a flow-through process according to the invention is used for separating an encapsulated virus from one or more contaminants. Exemplary encapsulated viruses include, but are not limited to, an influenza virus (e.g., an H1N1 strain), an adenovirus and a bacteriophage.

In some embodiments, the large biomolecule is produced in a cell culture or in eggs. In some embodiments, the cell culture comprises MDCK cells. In some embodiments, the sample comprising a large biomolecule and one or more contaminants is a cell culture supernatant, which is subjected to a flow-through process according to the present invention.

In various embodiments of the flow-through process according to the present invention, at least one population of solid porous particle include in the process comprises an average diameter ranging from 200 $\mu$m through 600 $\mu$m.

In various embodiments, a flow-through process of the present invention enables a recovery of at least 50%, or at least 55%, or at least 60%, or at least 65%, or at least 70%, or at least 75%, or at least 80%, or at least 85%, or at least 90% of a large biomolecule from a sample.

## Brief Description of the Drawings

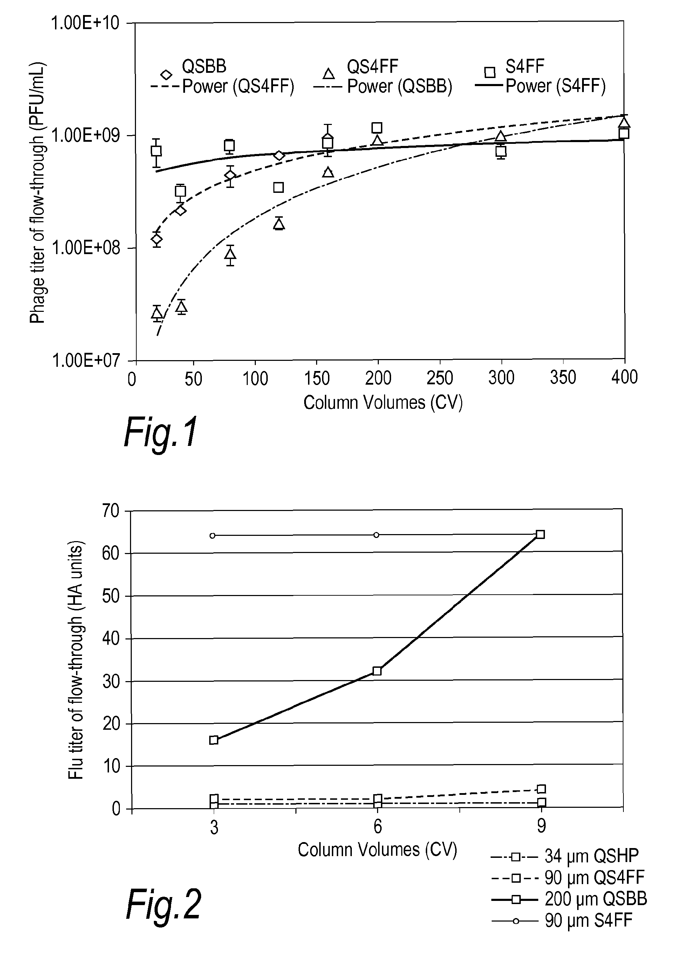

Figure 1 is a graph depicting the results of an exemplary experiment to measure the flow-through titer of bacteriophage Phi6 in a solution mixture of Phi6 and BSA passing through 0.5 ml columns, containing one of: S4FF (control) beads; QS4FF (90 $\mu$m) beads; or QSBB (200 $\mu$m) beads. The X-axis depicts the column volumes of feed processed through each column and the Y-axis depicts the Phi6 titer in the flow-through from each column.

Figure 2 is a graph depicting the results of an exemplary experiment to measure the flow-through titer of influenza virus-type B in a solution mixture containing influenza virus-type B, host cell DNA, host cell proteins, fetal bovine serum and other cell culture additives passed through 1ml columns, containing one of: QSepharose HP (30 $\mu$m) beads; QSepharose 4FF (90 $\mu$m) beads; or QSepharose BB (200 $\mu$m) beads. The X-axis depicts the column volumes of feed processed through each column and the Y-axis depicts the titer of influenza virus-type B in the flow-through from each column.

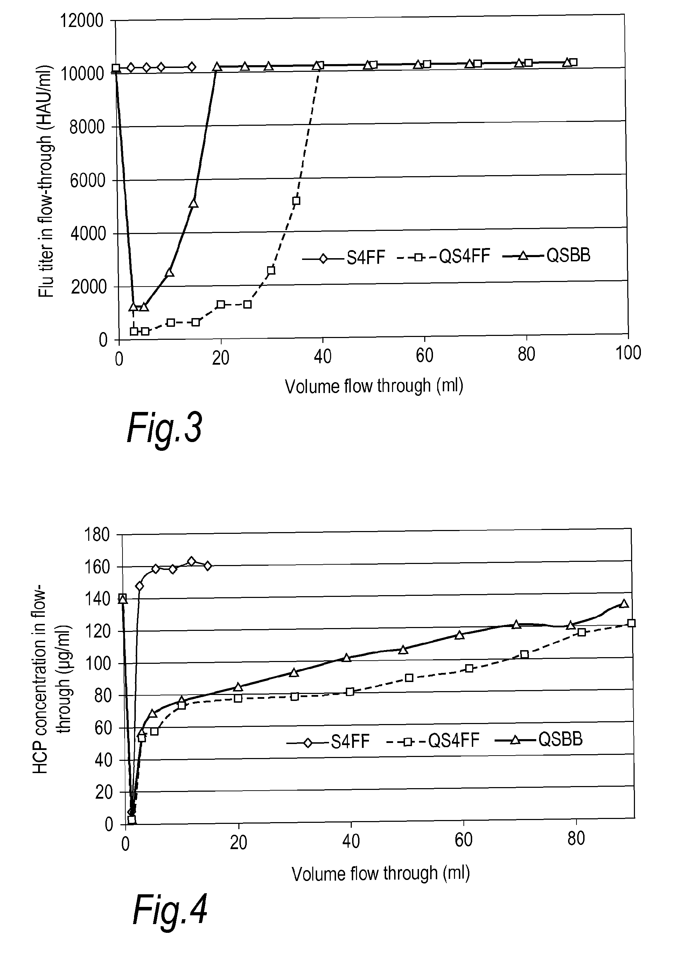

Figure 3 is a graph depicting the results of an exemplary experiment measuring the breakthrough of influenza virus type A in a mixture of host cell protein and DNA, passing through 0.8 ml columns containing one of: S4FF (control) beads, QS4FF (90 $\mu$m)beads; or QSBB (200 $\mu$m) beads. The X-axis depicts the volume of feed processed through each column and the Y-axis depicts the titer of influenza virus-type A in the flow-through from each column.

Figure 4 is a graph depicting the results of an exemplary experiment measuring the breakthrough of host cell protein (HCP) in a solution mixture of influenza virus type A and DNA, passing through 0.8 ml columns containing one of: S4FF (control) beads; QS4FF (90 μm)beads; or QSBB (200 μm) beads. The X-axis depicts the volume of feed processed through each column and the Y-axis depicts the titer of HCP in the flow-through from each column.

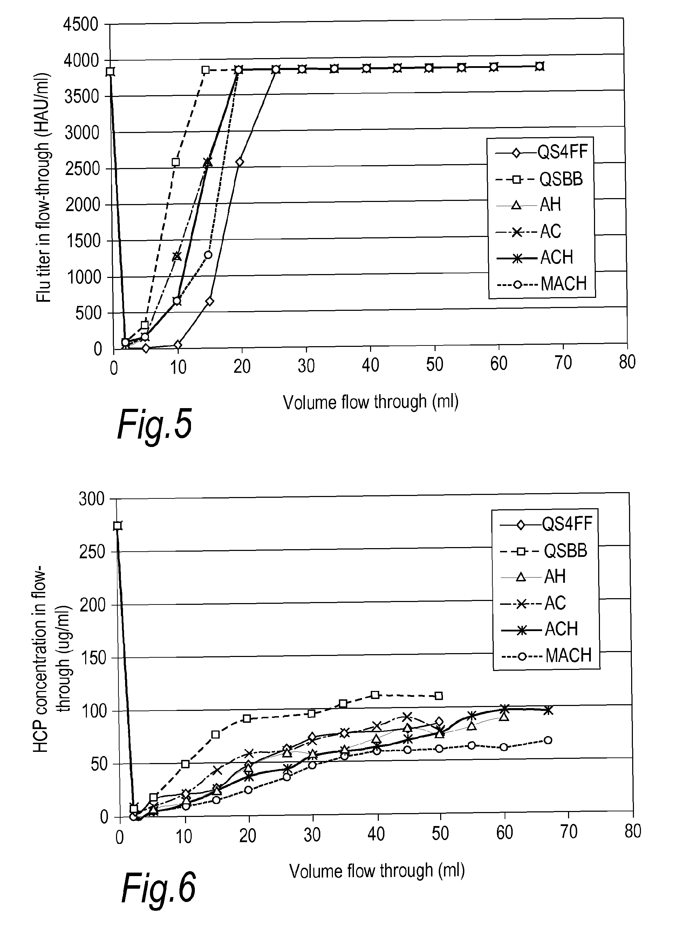

Figure 5 is a graph depicting the results of an exemplary experiment measuring the breakthrough of influenza virus type A in a solution mixture of HCP and host cell DNA, passing through columns containing one of: 0.8 ml of QS4FF (90 μm) beads; 0.8 ml of QSBB (200 μm) beads; 0.8 ml of QSBB and 0.8 ml of SP Sepharose big beads (200 μm) in series (AC); 0.8 ml of QSBB and 0.8 ml of Phenyl Sepharose big beads (200 μm) in series (AH); 0.8ml of QSBB, 0.8 ml of SP Sepharose big beads and 0.8 ml of Phenyl Sepharose big beads in series (ACH); or 2.4 ml of a mixture of QSBB, SP Sepharose big beads and Phenyl Sepharose big beads (MACH). The X-axis depicts the volume of feed processed through each column and the Y-axis depicts the titer of influenza virus type B in the flow-through from each column.

Figure 6 is a graph depicting the results of an exemplary experiment measuring the breakthrough of HCP in a solution mixture of influenza virus type A and host cell DNA, passing through columns containing one of: 0.8ml of QS4FF (90 μm) beads; 0.8ml of QSBB (200 μm) beads; 0.8 ml of QSBB and 0.8 ml of SP Sepharose big beads (200 μm) in series (AC); 0.8 ml of QSBB and 0.8 ml of Phenyl Sepharose big beads (200 μm) in series (AH); 0.8ml of QSBB, 0.8 ml of SP Sepharose big beads and 0.8 ml of Phenyl Sepharose big beads in series (ACH); or 2.4 ml of a mixture of QSBB, SP Sepharose big beads and Phenyl Sepharose big beads (MACH). The X-axis depicts the volume of feed processed through each column and the Y-axis depicts the titer of HCP in the flow-through from each column.

## Detailed Description of the Invention

The present invention provides an improved flow-through process for separating a large biomolecule (e.g., an encapsulated virus, a virus-like particle or a conjugate vaccine) from one or more contaminants in a sample using at least one type of chromatographic bead comprising an external surface area per unit volume which is minimized and an internal surface are per unit volume which is not decreased by more than 25%, relative to a similar type of chromatographic bead, which does not comprise a minimized external surface area per unit volume and an internal surface area per unit volume which is not decreased.

## I. Definitions

In order that the present disclosure may be more readily understood, certain terms are first defined. Additional definitions are set forth throughout the detailed description.

The terms "flow-through process," "flow-through mode," and "flow-through chromatography," as used interchangeably herein, refer to a product separation technique in which at least one product (e.g., a large biomolecule such as that described herein) contained in a sample along with one or more contaminants is intended to flow through a chromatographic resin or media, while at least one potential contaminant or impurity binds to the chromatographic resin or media. The "flow-through mode" is generally an isocratic operation (i. e., a chromatography process during which the composition of the mobile phase is not changed). Such flow-though chromatography processes can be operated using single columns, multiple columns in series or parallel, tandem columns, simulated moving beds (SMB) and combinations there of.

The terms "bind and elute mode" and "bind and elute process," as used interchangeably herein, refer to a product separation technique in which at least one product contained in a sample (e.g., a large biomolecule such as that described herein) binds to a chromatographic resin or media and is subsequently eluted.

The terms "contaminant," "impurity," and "debris," as used interchangeably herein, refer to any foreign or objectionable molecule, including a biological macromolecule such as a DNA, an RNA, one or more host cell proteins, endotoxins, lipids and one or more additives which may be present in a sample containing the large biomolecule of interest that is being separated from one or more of the foreign or objectionable molecules using a flow-through process of the present invention. Additionally, such a contaminant may include any reagent which is used in a step which may occur prior to the separation process. Generally, the contaminants present in a sample containing the large biomolecule are smaller in size than the large biomolecule. For example, most host cell proteins range from about 3 KD to 150 KD, which is smaller than the size of most large biomolecules to be separated using the methods described herein.

The term "large biomolecule" as used herein, refers to a biological material of interest having an average diameter of at least 15 nm, or at least 20 nm, or at least 25 nm, or at least 30 nm, or at least 35 nm, or at least 40 nm, or at least 45 nm, or at least 50 nm, or at least 55 nm, or at least 60 nm, or at least 65 nm, or at least 70 nm, or at least 75 nm, or at least 80 nm, or at least 90 nm, or at least 100 nm, or at least 110 nm, or at least 120 nm, or at least 130 nm, or at least 140 nm, or at least 150 nm, or at least 160 nm, or at least 170 nm, or at least 180 nm, or at least 190 nm, or at least 200 nm, or at least 210 nm, or at least 220 nm, or at least 230 nm, or at least 240 nm, or at least 250 nm, or at least 260 nm, or at least 270 nm, or at least 280 nm, or at least 290 nm, or at least 300 nm, or at least 310 nm, or at least 320 nm, or at least 330 nm, or at least 340 nm, or at least 350 nm, or at least 360 nm, or at least 370 nm, or at least 380 nm, or at least 390 nm, or at least 400 nm, which is separated from one or more contaminants that are present in a sample containing the biological material, using the flow-through processes described herein. Exemplary large biomolecules include, for example, encapsulated viruses, virus-like particles and conjugate vaccines. In some embodiments, a large biomolecule being separated using the methods of the present invention is either excluded from the pores of a chromatography bead or binds only to the external surface of the bead.

The term "encapsulated virus," as used herein, is meant to refer to any virus which includes a nucleic acid molecule (e.g., a DNA or an RNA) inside a capsid and generally only binds to the external surface of a chromatographic bead. In some embodiments, the capsid has a lipid envelope around the capsid. In some embodiments, an encapsulated virus includes a RNA or a DNA molecule enclosed inside a complex membrane structure (e.g., the smallpox virus from the *Poxviridae* family). Generally, most encapsulated viruses have a size ranging from 15-400 nm.

The term "virus-like particle," as used herein, is meant to refer to a particle or a substance which resembles a virus with respect to its size and/or its immunogenicity, however, lacks the ability to replicate like a virus (e.g., a human papillomavirus (HPV) vaccine).

The term "conjugate vaccine," as used herein is meant to refer to a vaccine which is produced by reacting a bacterial cell surface polysaccharide with a protein or toxin. An exemplary conjugate vaccine includes, but is not limited to, a *Pneumococcal* vaccine and a *Streptococcal pneumoniase* vaccine.

The term "chromatography," as used herein, refers to any kind of technique which separates a large biomolecule of interest (e.g., an encapsulated virus, a virus-like particle or a conjugate vaccine) from one or more contaminants which are present in a sample along with the large biomolecule of interest.

The term "anion exchange chromatography" or "AEC," as used herein, refers to a chromatographic process that uses positively charged beads to separate a biomolecule of interest (e.g., a large biomolecule such as an encapsulated virus, a virus-like particle or a conjugate vaccine) from one or more contaminants in a sample. Exemplary commercially available AEC media include Q SepharoseFF, Q Sepharose XL, Capto Q, Q Sepharose HP, all from GE Healthcare, Fractogel EMD TMAE, Fractogel EMD DEAE from EMD Chemicals Inc, and Toyopearl DEAE, Toyopearl QAE and Toyopearl SuperQ from TOSH Bioscience.

Conventionally, AEC has been used for virus purification using beads, membranes and monoliths. AEC is generally based on the principle that encapsulated viruses such as, for example, influenza and adenovirus, are negatively charged at a pH of above 5.5. Accordingly, they can be bound and eluted using positively charged resin *via* ionic interactions at pH above 5.5. Generally, due to the size of most encapsulated viruses, they cannot penetrate most commercially available AEC media and can only bind to the external surface of the media, thereby limiting the virus capacity of a chromatography column. Accordingly, a common approach to circumvent the column capacity problem has been to decrease the size of the AEC media (see, e.g., PCT Publication No. WO2004112707).

The term "cation exchange chromatography" or "CEC," as used herein, refers to a chromatographic process that uses negatively charged beads to separate a biomolecule of interest (e.g., a large biomolecule such as an encapsulated virus, a virus-like particle or a conjugate vaccine) from one or more contaminants in a sample. Exemplary commercially available CEC media include SP Sepharose, SP Sepharose HP, all from GE Healthcare, Fractogel EMD $SO_3^-$ from EMD Chemicals Inc, and Toyopearl SP and Toyopearl CM from TOSH Bioscience.

The term "hydrophobic interaction chromatography" or "HIC," as used herein, refers to a chromatographic process that uses beads immobilized with aromatic or aliphatic hydrocarbons as hydrophobic groups to separate a biomolecule of interest. In one embodiment, HIC is used for separating a large biomolecule such as, for example, a conjugate vaccine from one or more contaminants in a sample. Exemplary commercially available HIC media include Phenyl Sepharose from GE Healthcare, Fractogel EMD Propyl 650, Fractogel EMD Phenyl 650 from EMD Chemicals Inc, and Toyoperal Phenyl, Toyopearl Butyl, Toyopearl Hexyl and Toyopearl Ether from TOSH Bioscience.

The term "size exclusion chromatography" or "SEC," as used herein, refers to a chromatography process which is based on the principle that, when a solution containing solutes of different sizes is passed through a porous media having an appropriate pore size, the smaller size solutes take a longer time to diffuse out of the media and therefore have a longer retention time, whereas, the larger size solutes diffuse out quickly, thereby allowing their separation from the smaller size solutes. Exemplary commercially available SEC media include Toyopearl HW, Sepharose, Sephacryl and Fractogel EMD BioSEC.

Due to the size of most viruses, they cannot penetrate most commercially available SEC media and are typically eluted in the void volume of a SEC chromatography column. However, SEC generally has several disadvantages, for example, typically SEC chromatography requires long column length, the maximum loading capacity cannot exceed 20%, the processing times are very long and often results in dilution of the product of interest.

The term "mixed mode chromatography," or "bimodal chromatography" or "multimodal chromatography," as used interchangeably herein, refers to a chromatographic process that uses beads that have more than one functional group and/or a combination of various functional groups on a single bead. Such beads may be referred to as mixed mode resin or beads, bimodal resin or beads or multimodal resin or beads. For example, a single bead may have both positive and negatively charged groups or both positively charged and hydrophobic groups or both negatively charged and hydrophobic groups or each of positive, negative and hydrophobic groups on the same bead. Such beads can be used to separate a biomolecule of interest from one or more impurities in a sample. Exemplary commercially available mixed mode media include, e.g., Capto adhere from GE healthcare, HPA and PPA HyperCel™ from Pall Corporation,

The term "dynamic binding capacity" of a chromatography media, as used herein, refers to the amount of a target biomolecule the media binds in a flow-through process before a significant breakthrough of unbound biomolecule occurs. The dynamic capacity of a chromatography media reflects the impact of mass transfer limitations that may occur as flow rate is increased and it is useful in predicting real process performance compared to a determination of saturated or static binding capacity of the media. Generally dynamic binding capacity of a media is lower than the static binding capacity.

The terms "static binding capacity" and "saturated binding capacity," of a chromatography media, as used interchangeably herein, refer to the amount of a target biomolecule the media (e.g., beads packed in a column) can bind under static equilibrium conditions if every available binding site on the beads is utilized. The static binding capacity can be determined, for example, by loading a large excess of a target biomolecule either at very slow flow rates or after prolonged incubation in a closed system of beads in a column. In practice, the static binding capacity is never available under process conditions using packed columns.

The term "external surface area per unit volume," as used herein, refers to the total area on the external surface of a population of chromatography particles (e.g., beads) which is available for binding a biomolecule of interest. For example, in some embodiments, the external surface area per unit volume is defined as the total area on the external surface of a population of solid porous particles packed in a column, which is available for binding a large biomolecule.

An estimate of the external surface area per unit volume of beads can be calculated either mathematically or empirically. For example, mathematically, the external surface area per unit volume may be estimated using the following equation,

$$A_{es/V} = \frac{6\eta}{d} \quad \text{(Equation I)}$$

Where, $A_{es/v}$ = external surface area per unit volume; d = diameter of the bead used; and $\eta$ = packing density of the column. For a well packed column this is equal to 0.74, assuming hexagonal closed packing of beads (HCP).

Also $\eta$ = 1- void volume. Void volume of a column can be determined experimentally using a molecule which does not interact with the beads and is excluded by the pores of the beads (see, e.g., Gel filtration-Principles and Methods, Amersham Biosciences).

Empirically, the external surface area per unit volume is estimated as follows. Typically, the empirical calculation is performed using spherical nanoparticle of the same size and charge as the large biomolecule. The concentration of the nanoparticles obtained is usually specified by the manufacturer or can be obtained using a standard techniques used for determination of viral particles, such as dynamic light scattering or UV-Vis spectroscopy (see, e.g., Wolfgang Haiss et al., Determination of Size and Concentration of Gold Nanoparticles from UV-Vis Spectra, 2007, 79, 4215-4221). The binding of the nanoparticles to a fixed volume of beads is studied using static binding capacity experiments described herein, for example. The amount of nanoparticles bound to the bead can be estimated using the following formula,

$$N_n = (C_{in} - C_f)v \quad \text{(Equation II)}$$

Where, $N_s$ = amount of nanoparticle bound to beads at saturation; $C_{in}$ = initial concentration of nanoparticles in solution measured as particles per unit volume; $C_f$ = final concentration of nanoparticles in solution measured as particles per unit volume; and v = volume of solution used.

The external surface area per unit volume of a column containing the nanoparticles can be determined as follows,

$$A_{es/v} = N_n \times \frac{\pi d_n^2}{4} \times \frac{1}{V} \quad \text{(Equation III)}$$

Where, $d_n$ = diameter of nanoparticle; and V = volume of column.

The term "internal surface area per unit volume," of a population of chromatography particles (e.g., beads), as used herein, can be defined as the total surface area per unit volume of the particles minus the external surface area per unit volume of the particles and can be estimated as follows:

$$A_{in/v} = A_t - A_{es/v} \text{ (Equation IV)}$$

Where, $A_{in/v}$ = internal surface area per unit volume of beads; At = total surface area of the beads per unit volume; and At = A x BD, where A = surface area per unit mass of bead, which can be calculated using the method developed by Brunauer, Emmett, and Teller (BET) (see, e.g., S. Brunauer et al., J. Am. Chem. Soc., 1938, 60, 309); and BD = bulk density, which has the units of mass per unit volume. Generally, $A_{es/v}$ is negligible compared to At. Therefore, At can be considered to be a direct estimate of the internal surface area

An estimate of the internal surface area per unit volume can also be obtained from the static and dynamic binding capacities of the beads for small molecules representative of the size and charge of the host cell protein and other impurities in the feed stream. Example of such representative molecules may include but are not limited to bovine serum albumin (BSA), lysozyme or monoclonal antibodies (Mab, or IgG). For all practical purposes this method is a more realistic approach of estimating the surface area available for binding impurities.

The percent recovery of a product of interest in a flow-through process can be calculated using the following equation,

$$\% \text{ Recovery} = \frac{P_{feed} - P_{flow-through}}{P_{feed}} \times 100 \text{ (Equation V)}$$

Where, $P_{feed}$ = amount of large biomolecule in feed and $P_{flow-through}$ = amount of large biomolecule in product.

The percent recovery of a product of interest using the methods according to the present invention can be calculated using the following equation, where the percent recovery is percent improved recovery:

$$\% \text{ Improved Recovery} = \frac{R_b - R_s}{R_b} \times 100 \text{ (Equation VI)}$$

Where $R_b$ = recovery from a process in which the external surface area per unit volume of a population of particles has been minimized; and $R_s$ = recovery from a process in which the external surface area per unit volume of the population of particles has not been minimized.

Purity of the product recovered using the flow-through process can be defined as the ratio of the amount of impurities to the large biomolecule in the recovered flow-through pool. For example, the purity of an influenza virus containing product can be expressed as μg/HAU (HAU= hemaglutinin units), which is obtained by dividing the total amount of impurities (e.g., host cell proteins) by the total amount of virus in the flow-through pool. Percent improvement in purity of the product of interest using the methods according to the present invention can be calculated using the following equation,

$$\% \text{ Improved purity} = \frac{r_s - r_b}{r_s} \times 100 \text{ (Equation VII)}$$

Where, $r_b$ = ratio of the total amount of host cell protein in the product to the total amount of the large biomolecule in the product, when a process with a population of particles (e.g., solid porous beads) having a minimized external surface area per unit volume is used. For example, for influenza purification this ratio can be expressed in μg/HAU, where μg is the unit for the host cell protein and HAU represents the units for influenza virus: and $r_s$ = ratio of the total amount of host cell protein in the product to the total amount of the large biomolecule in the product, when a process in which a population of particles (e.g., solid porous beads) having a minimized external surface area per unit volume are not used.

In general, particles having a minimized external surface area per unit volume used in the claimed methods have an internal surface area per unit volume which is not decreased by more than 25% relative to the particles which do not have a minimized external surface area per unit volume.

## II. Exemplary chromatography media used in flow-through process

Exemplary chromatography media that may be used for flow-through purification include, but are not limited to, anion exchange chromatography media (e.g., Q Sepharose Big Beads, Q SepharoseFF, Q Sepharose XL, Capto Q, Q Sepharose HP, Fractogel EMD TMAE, Fractogel EMD DEAE, Toyopearl DEAE, Toyopearl QAE and Toyopearl SuperQ), cation exchange chromatography media (e.g., SP Sepharose Big Beads, SP Sepharose, Fractogel EMD SO$_3^-$, SP Sepharose HP, Toyopearl SP and Toyopearl CM), hydrophobic interaction chromatography media (e.g., Phenyl Sepharose, Toyoperal Phenyl, Toyopearl Butyl, Toyopearl Hexyl and Toyopearl Ether) and size exclusion chromatography media (*e.g.*, Toyopearl HW, Sepharose, Sephacryl and Fractogel EMD BioSEC). A single type of chromatography media or any combination of one or more types of chromatography media may be used in the methods of the present invention. It would be readily apparent to one of ordinary skill in the art as to which chromatography media may be used in the methods of the present invention, largely on the basis of the large biomolecule being separated and the impurity required to be removed.

## III. Flow-through methods of separating biomolecules from one or more contaminants

Flow-through chromatographic separation has become an important final polishing step for removing trace impurities such as, for example, endotoxins, viruses and DNA from monoclonal antibody preparations. Flow-through separation has advantages over conventional bind and elute chromatography mode because it eliminates the need for complicated steps during the chromatographic separation process such as buffer change, gradients and peak separation.

Flow-through chromatography methods of separation generally involve contacting a feed solution containing a biomolecule of interest and one or more impurities with a media such as beads or membrane and collecting the treated feed. In some flow-through processes, the media is packed inside a flow-through device such a column. In some processes, such a device contains media (e.g., solid porous beads) that can capture most of the impurities in the feed stream, with minimal to no binding for the product of interest, consequently, resulting in a purer product downstream of the flow-through device.

In some embodiments according to the present invention, the flow-through device consists of a column packed with one or more types of chromatography beads, such as those known in the art and/or described herein, where at least one type of bead comprises a minimized external surface area per unit volume of the beads and an internal surface area per unit volume of the beads which is not decreased by more than 25% relative to the beads not having a minimized external surface area.

In some embodiments, a feed containing a large biomolecule such as, for example, an encapsulated virus (e.g., influenza virus), a virus-like particle (e.g., bovine serum albumin (BSA) coated styrene particle) or a conjugate vaccine is contacted with one or more types of chromatography beads, each having a molecular weight cut off smaller than the biomolecule, in order to separate the large biomolecule from one or more contaminants in the feed, where at least one type of the beads have a minimized external surface area per unit volume of the one type of beads and an internal surface area per unit volume of the one type of beads, which is not decreased by more than 25% relative to the internal surface area per unit volume of the same type of beads which does not have a minimized external surface area per unit volume of the beads.

For example, in an exemplary experiment described herein, the influenza virus was grown using a MDCK cell line. The virus-containing feed was preclarified using centrifugation followed by use of a 0.45 $\mu$m membrane filtration in order to remove large cell debris and then buffer exchanged into the desired buffer before purification through a flow-through device containing the chromatography beads. In general, one or more of preparative steps such as, for example, centrifugation, depth filtration, tangential flow filtration, dia- filtration and buffer exchange may be used for various feeds containing viruses obtained from different sources (e.g., cell culture, eggs *etc.*) before purification using the flow-through chromatography mode, as described herein.

Following preclarification of a feed containing the biomolecule of interest, the preclarified feed is subjected to flow-through separation. Flow-through separation may be performed using one or more setups. In an exemplary setup, a positive displacement pump (e.g., Mighty-Mini, Scientific Systems Inc.) and a flow-through column are used in series. For example, the pump and the tubing are first sanitized using 20% ethanol and then flushed with water and buffer. A feed containing the large biomolecule of interest is subsequently passed through the column using the pump and fractions are collected manually downstream of the column into eppendorf tubes.

In another exemplary setup, a more self contained apparatus such as an Akta (GE Healthcare) or a BioCad (Applied Biosystems) may be used. These systems allow for automated pumping and control of feed and buffers, inline measurement of UV and conductivity signals of the flow-through streams and automatic fraction collection. The fractions collected are further used for offline measurements.

## IV. Methods of measuring titer of recovered biomolecule in a flow-through Process

Following the separation of a large biomolecule from one or more contaminants in a sample, the titer of the recovered large biomolecule is generally measured.

Techniques for the detection of various biomolecules including viruses, proteins, nucleic acids, conjugate vaccines *etc.* are generally well known in the art and have been described in the literature. Titers of biomolecules recovered using chromatography processes may be measured either inline or offline. An inline detection technique involves detectors in series placed downstream of the chromatographic column. Whereas offline methods of analysis are accomplished by collecting fractions of the solution flowing through the chromatography column and assaying those using standard techniques. Offline detection is generally used if there are interferences from different species in the inline technique, if the biomolecule is not detectable using an available inline technique or if concentrations of a biomolecule of interest are below the detection limits of the inline technique or not representative of the actual concentrations.

A commonly used inline measurement technique for viruses, proteins and DNA is UV spectroscopy at 280 nm and 260 nm (see, e.g., European patent application no. EP 1801 697 A1). Also, recently multi-angle laser light scattering (MALS) has been successfully used to detect viruses inline in a chromatographic process (see, *e.g.,* Opitz et al., Biotech. Bioengg., 103:1144-1154 (2009)).

Offline measurement techniques include the above mentioned techniques, such as UV spectroscopy and dynamic light scattering (see, *e.g.,* Opitz et al., J. Biotech., 131:309-317 (2007)) as well as additional techniques that are unique to the biomolecule of interest which is being measured. For example, some of the commonly used offline measurement techniques for proteins include, the bicinchoninic acid assay (BCA) (see, *e.g.,* U.S. Publication no. 20050118140), the Bradford assay (see, *e.g.,* Nayak et al., J. Chrom., 823:75-81 (2005)) and the Enzyme-Linked Immunosorbent Assay (ELISA) (see, e.g., U.S. Publication no. 20050118140).

Similarly, nucleic acids (e.g., DNA) can be measured using the picogreen assay (see, *e.g.,* Opitz et al., Biotech. Bioengg., 103:1144-1154 (2009)) or quantitative polymerase chain reaction (Q-PCR).

Most viruses can be detected and quantified using a tissue culture infectious dose assay (also referred to as TCID50 plaque assay) (see, e.g., Dulbecco et al., Cold Spring Harbor Symp. Quant. Biol., 18:273-279 (1953)) However, unique assays are generally used to identify and quantify particular viruses, e.g., the infuenza virus is quantified using assays such as hemagglutination (HA), neuraminidase (NA) and single radial immunodiffusion (SRID) assays (see, e.g., Methods in Molecular Biology 436, Avian Influenza Virus, edited by Spackman, E. Humana Press (2007)).

## V. Methods of measuring sizes of chromatographic beads and viruses

In some embodiments, the chromatography methods of present invention employ one or more types of solid porous chromatography beads, where ant least one bead type has an average diameter which is larger than the average diameter of the other bead types. Such a bead is referred to as a "big bead" or a "large bead" in the context of the present invention. In some embodiments, a big bead has an average diameter of at least 200 $\mu$m.

Based on the type of the biomolecule being separated using the methods of the present invention, one of ordinary skill in the art can readily determine one or more types of chromatography beads that may be required for the separation of the biomolecule (e.g., based on the composition, size, charge *etc.* of the biomolecule).

In general, most chromatography beads used in the methods of the present invention can be obtained commercially. Accordingly, the sizes of chromatographic beads are usually set forth in the manufacturers specification sheets or can be readily determined using one or more of methods or instruments which are well known in the art including but not limited to: (1) wet sieving which may also be used to segregate beads with a wide size distribution into narrow size ranges; (2) an optical microscope with an inbuilt camera and micro meter slide or by taking a picture of the beads and analyzing them using software such as ImageJ; and (3) using standard particle sizers such as Elzone II (Particle and Surface Sciences Pty. Ltd), Master Sizer (Malvern) and FlowCam (Fluid Imaging Technologies).

Sizes of most large biomolecules (e.g., viruses, virus-like particles and conjugate vaccines) which may be separated using the methods of the present invention can generally be determined based on the methods that are well characterized in the art. For example, the size of a virus can be determined using one or more techniques that are well known in the art, including, but not limited to, scanning electron microscopy (SEM) (see, e.g., Nermut et al., AIDS Res., 9: 929-938 (1993) and dynamic light scattering (Dynopro, Wyatt Technology Corporation) (see, *e.g.,* Opitz et al., J. Biotech. 131, 309-317 (2007)). An estimate of the size of the large biomolecule can be obtained by using neutral beads that have no interactions with the large biomolecule and a pore size similar to that of the beads being used in the flow-through purification. If the large biomolecule is larger than the pores of the beads it will be eluted in the void volume of the column of such beads. This will provide an estimate of whether the size of the large biomolecule is greater than the pore size of the beads.

## VI. Methods of measuring pore sizes of chromatographic beads

Pore sizes, which are indicative of the molecular weight cut offs of chromatographic beads, are generally specified by manufacturers. These have also been widely discussed in the literature (see, *e.g.,* Gu et al., Enzy. Micro. Tech. 33:430-437 (2003)). Common techniques used to measure pore sizes of beads include, but are not limited, to: (1) Dextran exclusion chromatography (see, *e.g.,* DePhillips et. al., J. Chrom. A. 883:39-54 (2003)); (2) Porosimetry generally using mercury (see, *e.g.,* Sobisch et al., Colloid Poly. Sci. 133:169-172 (2006)); (3) SEM (4) Gas sorption (see, e.g., Brunauer et al., J. Am. Chem. Soc., 60:309-319 (1938)); and (5) Thermoporometry (see, *e.g.,* Wulff et al., Therchimi. Acta, 419: 291-294 (2004)).

This invention is further illustrated by the following examples which should not be construed as limiting. The contents of all references, patents and published patent applications cited throughout this application, as well as the Figures, are incorporated herein by reference.

## Examples

### Example 1: Determination of external surface area of beads per unit volume

The external surface area of beads of different sizes per unit volume of the beads can be determined as described herein. In an exemplary experiment, the external surface area of the following beads per unit volume was estimated using the mathematical method described above, Q Sepharose HP with an average mean diameter of 34 um, Q Sepharose FF with an average mean diameter of 90 $\mu$m and Q Sepharose BB with an average mean diameter of 200 $\mu$m (GE Healthcare).

For example, using equation I discussed herein, the external surface area per unit volume for a 34 $\mu$m average diameter bead can be calculated as follows:

$$A_{es/V} = \frac{6\eta}{d} = \frac{6*0.74}{34 \times 10^{-4}} = 1305 cm^{-1}$$

Similarly the external surface area for all the other AEC beads was calculated and is summarized in Table I.

**Table I:**

| Type of Bead | Bead Size ($\mu$m) | $A_{es/v}$ (cm$^{-1}$) |
|---|---|---|
| Q Sepharose HP | 34 | 1305 |
| Q Sepharose FF | 90 | 493 |
| Q Sepharose BB | 200 | 222 |

As can be observed from Table I, the external surface area decreases with increase in bead size.

In another exemplary experiment, the empirical method described herein was used for the calculating of the external surface area per unit volume of beads. An approximate estimate of the external surface area available on Q Sepharose FF (90$\mu$m) and Q Sepharose BB (200$\mu$m) for binding large biomolecules such as influenza or adenovirus was obtained empirically using BSA coated latex beads (Postnova Analytics) as a model.

5 ml columns of each of Sepharose 4FF (S4FF, GE Healthcare), QS4FF or QSBB beads were transferred to a 7 ml tube having a lid. S4FF is neutrally charged and was used as a control bead. About 1.38 ml of BSA coated styrene particle solution (1.4 x 10$^{13}$ particles/ml) in PBS was added to the each of the beads.

The bead-particle mixture was mixed overnight in a rotating motion, the beads were allowed to settle and absorbance of the particles in the supernatant was measured using UV spectroscopy at 280 nm. The concentration of the particles was derived by plotting a standard calibration curve.

The results of an exemplary experiment measuring the surface area using BSA coated styrene particles using equation III set forth above for each of the aforementioned beads is summarized in Table II.

**Table II:**

| Sample ID | Bead size (μm) | Supernatant Conc. (mg/ml) | BSA adsorbed (mg/ml) | $A_{es/v}$ (CM$^{-1}$) |
|---|---|---|---|---|
| Initial | | $1.38 \times 10^{13}$ | | |
| S4FF* | 90 | $5.88 \times 10^{12}$ | 0 | 0 |
| QS4FF | 90 | $3.45 \times 10^{12}$ | $6.7 \times 10^{11}$ | 52 |
| QSBB | 200 | $5.16 \times 10^{12}$ | $1.9 \times 10^{11}$ | 15 |
| *Seph-4FF represents control for void volume dilution of feed. Data shown accounts for the dilution of the feed caused by the buffer in the void volume of the beads | | | | |

As observed herein, the external surface area per unit volume calculated both empirically as well as mathematically decreases with an increase in bead size. However the empirical values suggest that the actual area available for binding the large biomolecule is much lower than the mathematically calculated area. In the flow-through methods described herein, it is desirable to use at least one population of beads having an increased average diameter and having a minimal external surface area per unit volume. Accordingly, the external surface area of a population of beads is minimized by increasing the bead size, without decreasing the internal surface area per unit volume.

**Example 2: <u>Estimating internal surface area of beads per unit volume using static binding capacity</u>**

The internal surface area of QS4FF (90 μm) and QSBB (200μm) beads per unit volume, discussed in Example 1, having larger and minimized external surface area, respectively, was estimated by testing their static binding capacity for BSA. BSA has been widely used as a model protein in chromatography. It has an average molecular weight of approximately 66 KD and a pI of ~5. BSA is representative of the size and charge of most host cell proteins.

In one exemplary experiment, the static capacity of QS4FF and QSBB beads for BSA (Sigma Aldrich Corporation) was measured in two different types of buffers at two different pHs. The experiment was performed by packing a 1 ml column containing either QS4FF or QSBB media, slurring the media in 4 ml of buffer and adding 0.5 ml of the slurry into 14.5 ml of BSA solution having a concentration of 2 mg/ml. After 4 hours, the beads in the mixture were allowed to settle and concentration of the BSA in the supernatant was measured by UV-Vis spectroscopy. The change in the concentration of the supernatant was used to calculate the static binding capacity of the beads for the BSA.

The results of the experiment measuring the static capacity of the beads for BSA in different solutions are summarized in Table III.

**Table III:**

| Buffer | pH | BSA Capacity (mg/ml) | | |
|---|---|---|---|---|
| | | S4FF* (90μm) | QSBB (200μm) | QS4FF (90μm) |
| Tris 50mM | 8 | 0 | $43 \pm 2$ | $54 \pm 1$ |
| Tris 50mM | 7 | NA | $38 \pm 2$ | $38 \pm 1.5$ |
| Phos 50mM | 7 | NA | $13 \pm 0.1$ | $11.6 \pm 1.2$ |
| *S4FF are neutral sepharose beads which were used as control for the experiment. Tris and Phos stand for Trizma and sodium phosphate buffer respectively. | | | | |

As shown in Table III, the 200 μm beads with minimized external surface area and the 90 μm beads, have similar BSA capacity in Trizma and sodium phosphate buffer at pH 7.0. There is a slight decrease in the capacity of the QSBB (~20%) at pH 8.0 in Trizma buffer, however, the capacity at pH 8.0 is higher than at pH 7.0. Accordingly, it can be estimated that the QS4FF and QSBB beads with higher and lower external surface area per unit volume, respectively, have similar internal surface area per unit volume for binding BSA. Thus it can be concluded that by choosing the correct buffer, pH and bead sizes, similar quantities of impurities can be removed by using the method proposed in this invention.

**Example 3: Comparison of the recovery of virus-like particles (*i.e.,* 100 nm BSA coated styrene particles) from anion exchange media of two different bead sizes.**

In an exemplary experiment, two separate anion exchange media having different bead sizes were tested for the recovery of virus-like particles. Specifically, the static binding capacities of anion exchange media, Q Sepharose 4FF beads having an average diameter of 90 μm (QS4FF, GE Healthcare) and Q Sepharose Big beads having an average diameter of 200 μm (QSBB, GE Healthcare), were tested for the recovery of 100 nm Bovine serum albumin (BSA) coated styrene particles (Postnova Analytics), which are exemplary virus-like particles and have a similar size and charge as influenza or adenovirus. QS4FF and QSBB media contain quaternary ammonium groups which are positively charged and can bind BSA coated latex particles. Generally, it was assumed that the static binding capacities of these beads for the BSA coated particles should be equal to the dynamic binding capacity because the particles bind only to the external surface area of the beads.

5 ml columns of each of Sepharose 4FF (S4FF, GE Healthcare), QS4FF and QSBB beads were transferred to a 7 ml tube having a lid. S4FF is neutrally charged and was used as a control bead. About 1.38 ml of BSA coated styrene particle solution (25 mg/ml, $1.4 \times 10^{13}$ particles/ml) in PBS was added to the each of the beads.

The bead/particle mixture was rotated overnight, the beads were allowed to settle and absorbance of the particles in the supernatant was measured using UV spectroscopy at 280 nm. The concentration of the particles was derived by plotting a standard calibration curve.

The results of an exemplary experiment measuring the capacity for BSA coated styrene particles for each of the media and the concentration of the particles in the feed and the supernatant are summarized in Table IV.

**Table IV:**

| Sample No | Sample ID | Supernatant Conc. (mg/ml) | BSA-styrene adsorbed (mg) | BSA-styrene adsorbed (mg/ml) |
|---|---|---|---|---|
| 1 | Feed | 24.7 | | |
| 2 | S4FF | 10.5 | 14.2 | 2.8* |
| 3 | QS4FF | 6.2 | 18.6 | 3.7 |
| 4 | QSBB | 9.2 | 15.5 | 3.1 |
| * Seph-4FF represents control for void volume dilution of feed. | | | | |

Additionally, recovery of BSA coated styrene particles achieved with each of the beads is summarized in Table V.

**Table V:**

| Data shown accounts for the dilution of the feed caused by the buffer in the void volume of the beads. | | | | | | |
|---|---|---|---|---|---|---|
| Sample No | Sample ID | Bead size (μm) | Supernatant Conc. (mg/ml) | BSA adsorbed (mg) | BSA adsorbed (mg/ml) | % Normalized Recovery |
| 1 | Feed | | 24.7 | | | |
| 2 | S4FF | 90 | 10.5 | 0 | 0 | 100 |
| 3 | QS4FF | 90 | 6.1 | 4.3 | 0.87 | 58.7 |
| 4 | QSBB | 200 | 9.2 | 1.3 | 0.26 | 87.8 |

As shown in Table V, the recovery of BSA coated styrene particles increases by about 33% with increase in bead size. Accordingly, decreasing the external surface area per unit volume by increasing the bead size correlates with improved recovery of the virus-like particles.

**Example 4: Comparison of the recovery of bacteriophage-Phi6 from a mixture of Phi6 and BSA using anion exchange media of two different bead sizes.**

In another exemplary experiment, recovery of virus particles (Phi6) from a solution mixture containing Phi6 and BSA was demonstrated. Phi6 is a lipid enveloped bacteriophage with a size of about 86 nm and a negative charge at a pH above 7.0. Accordingly, it was used as a model to represent the influenza virus. Further, BSA is a negatively charged protein with a pI of about 5.0 and was used as a model to represent host cell protein. These studies were performed in dynamic mode by flowing solution through a packed column of beads at a flow rate of about 1 ml/min using the following procedure.

A feed solution was prepared by spiking bacteriophage Phi6 (stock titer of ~1.1x10$^{12}$ PFU/ml) into about 600 ml of PBS to achieve a final Phi6 concentration of about 109 PFU/ml. Bovine serum albumin (BSA, Sigma Aldrich Corporation) was added to this solution to achieve a final concentration of 0.18 mg/ml determined by UV spectroscopy.

Glass columns (Omnifit from Fisher Scientific Corporation) were packed with about 0.5 ml of neutral Sepharose 4FF having an average diameter of 90 $\mu$m (S4FF, GE Healthcare), positively charged Q Sepharose 4FF beads having an average diameter of 90 $\mu$m (QS4FF, GE Healthcare) or positively charged Q Sepharose Big beads having an average diameter of 200 $\mu$m (QSBB, GE Healthcare). The quality of the columns was verified using the acetone pulse method to ensure they had good asymmetry and HETP (*i.e.,* height equivalent of a theoretical plate) and then equilibrated in PBS (see, *e.g.,* as described in Ion Exchange Chromatography and Chromatofocusing- Principles and Methods. Amersham Biosciences, 11-0004-21, Edition AA).

Approximately 200 ml of feed solution was passed through each of the columns at a flow rate of 1 $\pm$ 0.1 ml/min and the flow-through was collected in 20 ml fractions.

The fractions were assayed for BSA concentration using UV spectroscopy at 280 nm using a Phi6 solution in buffer as control. The concentration of bacteriophage Phi6 was determined using TCID50-plaque forming unit assay.

The results of one such experiment measuring the breakthrough of bacteriophage Phi6 in a solution mixture of Phi6 and BSA, passed through 0.5 ml columns containing either S4FF (Control), QS4FF (90 $\mu$m) or QSBB (200 $\mu$m) beads is shown in Figure 1.

As evidenced by Figure 1, Phi6 passes through the control beads S4FF without any interactions, giving a flow-through concentration equal to the feed concentration. The flow-through using the QSBB beads (200 $\mu$m) suggests that the Phi6 initially binds to the column and reaches the feed concentration of within 150 column volumes (CV). However, the Phi6 concentration in the flow-through using the QS4FF beads (90 $\mu$m) reaches the feed concentration only after 300 CV. This suggests that the 200 $\mu$m beads bind only half as much virus as the 90 $\mu$m beads.

The results of one such experiment measuring the amount of BSA and Phi6 in the flow-through of a solution mixture containing the above passed through 0.5 ml columns containing either S4FF, QS4FF or QSBB at a particular point in time during the experiment is summarized in Table VI.

**Table VI:**

| Bead Type | Time (min) | BSA Concentration (mg/ml) | Phi6 Concentration (PFU/ml) |
|---|---|---|---|
| S4FF | 40 | 0.18 | 8 x 10$^8$ |
| QS4FF | 40 | 0.14 | 9 x 10$^7$ |
| QSBB | 40 | 0.14 | 4.45 x 10$^8$ |

Based on the results summarized in Table VI, it appears that even though the 90 $\mu$m and the 200 $\mu$m size beads bind different amounts of Phi6, they have similar binding characteristics for the BSA. Further, one log greater recovery of Phi6 can be obtained using QSBB (200 $\mu$m) as compared to QS4FF (90 $\mu$m) after passing about 80 CV of feed through each column.

Accordingly, based on the foregoing results, it appears that for a given column volume (CV), more virus can be recovered using bigger beads (e.g., 200 $\mu$m) relative to a smaller beads (e.g., 90 $\mu$m) due to decrease in external surface area per unit volume. Further, presence of proteins, e.g., host cell proteins, in the solution, does not appear to adversely affect the virus recovery and removal of the host cell proteins themselves appears independent of the bead size.

**Example 5: Comparison of the recovery of Influenza virus type B from a mixture of influenza virus type B and host cell protein using anion exchange media of three different bead sizes.**

In another exemplary experiment, the recovery of influenza virus-type B, from a mixture of host cell protein, DNA and other components in the growth medium such as fetal bovine serum (FBS), phenol red and non-essential amino acids is described. Influenza virus type B is a lipid enveloped virus with a pI of-5.0 and a size of approximately 80 to 120 nm. These studies were performed in dynamic mode by flowing clarified cell culture lysate supernatant containing influenza virus type B and host cell protein through a packed column of beads at a flow rate of 1 ml/min.

Approximately 200 ml of feed containing influenza virus type B grown in MDCK cells was prepared using standard procedure. Initially, eight T150 flasks were seeded with about 2.0 x 10^7 cells/flask in 10% FBS DMEM. After 24hrs, the media in the flasks was changed to 1% FBS in DMEM and the cells were infected with influenza virus type B strain B/Lee/40 (ATCC # VR-1535) which was tissue culture adapted to growth in MDCK cells. The flasks were then incubated at 37°C in 5% $CO_2$ for 5 days until complete CPE (cytopathic effect) was observed. The supernatant was subsequently centrifuged at 2500 RPM, filtered through a 0.45 $\mu$m membrane filter to remove cell debris and stored at 4°C before further use.

Glass columns (Omnifit from Fisher Scientific Corporation) were packed with about 1 ml of neutral Sepharose 4FF having an average diameter of 90 $\mu$m (S4FF, GE Healthcare), positively charged Q Sepharose HP having an average diameter of 34 $\mu$m (QSHP, GE Healthcare), positively charged Q Sepharose 4FF beads having an average diameter of 90 $\mu$m (QS4FF, GE Healthcare) or positively charged Q Sepharose Big beads having an average diameter of 200 $\mu$m (QSBB, GE Healthcare). The quality of the columns was verified using acetone pulse method to ensure they had good asymmetry and HETP and then equilibrated in 20 mM phosphate buffer pH 7.2.

Approximately 10 ml of clarified influenza feed, prepared as described above, was passed through each column at a flow rate of 1 ml/min. The flow through the columns was collected in 1 ml fractions and assayed in duplicate for flu content using hemagglutination (HA) assay.

The results of an exemplary experiment measuring the breakthrough titters of influenza virus type B in a solution mixture of host cell protein, DNA, FBS and other growth additives, passed through 1 ml columns containing either S4FF (Control), QSHP (34 $\mu$m) QS4FF (90 $\mu$m) or QSBB (200 $\mu$m) beads are shown in Table VII and Figure 2.

Table VII:

| sample type ↓ | HA units | | |
|---|---|---|---|
| CV fraction → | 3 | 6 | 9 |
| Flu hold | 64 | 64 | 64 |
| 90 $\mu$m S4FF | 64 | 64 | 64 |
| 34 $\mu$m QSHP | 1 | 1 | 1 |
| 90 $\mu$m QS4FF | 2 | 2 | 4 |
| 200 $\mu$m QSBB | 16 | 32 | 64 |
| Data is represented in hemagglutinin (HA) units. | | | |

Beads of three different sizes corresponding to three different external surface areas per unit volume were evaluated in this experiment. As can be observed in Table VII and Figure 2, the QSBB (200 $\mu$m) beads show instant break through of influenza virus type B (breakthrough at less than 3 CV), whereas the QSHP (34 $\mu$m) and do not breakthrough over the entire length of the experiment of upto 9 CV of feed passed through the columns. The flow-through concentration for the QSBB (200 $\mu$m) equals the feed solution concentration after 9 CV, giving 16 times greater recovery than the QS4FF (90 $\mu$m) beads and 64 times greater recovery than the QSHP (34 $\mu$m) beads. Accordingly, the results demonstrate that decreasing the external surface area per unit volume by increasing the size of the beads results in a higher recovery of influenza virus type B relative to smaller sized beads.

Further, Table VIII below summarizes the recovery of the various types of virus particles studied in the experiments described in Example 3, 4 and 5. The pool recoveries for bacteriophage-Phi6 and influenza virus type B was calculated using area under the break through curves in Figure 1 and Figure 2.

**Table VIII**

| Size of Anion Exchange Beady ↓ | % Recovery (pool) of Various Virus/Virus like Particles | | |
|---|---|---|---|
| | BSA coated Styrene Beads | Bacteriophage-Phi6 | Influenza B |
| 30 $\mu$m Q Sepharose HP | NA | NA | 1.5 |
| 90 $\mu$m Q Sepharose FF | 58.7 | 10 | 4 |
| 200 $\mu$m Q Sepharose BB | 87.8 | 100 | 56 |
| % Improved Recovery | 33 | 90 | 92-97 |

As summarized in Table VIII, minimizing the external surface area by increasing the size of the bead from 90 $\mu$m to 200 $\mu$m improved the yields of the viruses/virus like particles by 30-90%.

**Example 6: Comparison of the recovery of Influenza virus type A and removal of host cell protein from a mixture of influenza virus and host cell protein and DNA using anion exchange media.**

In another exemplary experiment, the recovery of influenza virus-type A, from a mixture of host cell protein and DNA was measured. Influenza virus type A is a lipid enveloped virus with a pI of ~5.0 and a size of approximately 80 to 120 nm. These experiments were performed in dynamic mode by flowing clarified and buffer exchanged feed containing influenza virus type A and host cell protein through a packed column of beads at a flow rate of 1 ml/min.

Feed containing influenza virus type A grown in MDCK cells was prepared using standard procedure. Initially, fifty T150 flasks were seeded at 10% confluency of MDCK cells in 10% FBS DMEM. After 2-3 days, once the cells were 80-90% confluent, the media in the flasks was changed to DMEM without serum and the cells were infected with influenza virus type A/WS (H1N1 strain) which was tissue culture adapted to growth in MDCK cells. The flasks were then incubated at 33°C in 5% $CO_2$ for 3 days until complete CPE was observed. The supernatant was subsequently centrifuged at 2500 RPM, filtered through a 0.45 $\mu$m membrane filter to remove cell debris and stored at -80°C before further use.

Glass columns (Omnifit from Fisher Scientific Corporation) were packed with about 0.8 ml of neutral Sepharose 4FF having an average diameter of 90 $\mu$m (S4FF, GE Healthcare), positively charged Q Sepharose 4FF beads having an average diameter of 90 $\mu$m (QS4FF, GE Healthcare) or positively charged Q Sepharose Big beads having an average diameter of 200 $\mu$m (QSBB, GE Healthcare). The quality of the columns was verified using acetone pulse method to ensure they had good asymmetry and HETP and then equilibrated in 50 mM phosphate buffer pH 7.1.

Just prior to conducting the experiment, approximately 200 ml of feed was thawed at 4°C overnight and then buffer exchanged into 50mM phosphate buffer at pH 7.1 using a 10 Kilo-Dalton Centricon centrifugal filter device (Millipore Corporation). Approximately 90 ml of this influenza feed, prepared as described above, was passed through each column at a flow rate of 0.16 ml/min. The flow through the columns was collected in 1 ml fractions and assayed in duplicate for flu, host cell proteins, and host cell DNA content using HA assay, BCA assay and Pico-green assay, respectively.

The results of an exemplary experiment measuring the breakthrough of influenza virus type A in a mixture of host cell protein and DNA, passing through 0.8 ml columns containing S4FF (Control), QS4FF (90 $\mu$m) or QSBB (200 $\mu$m) beads is shown in Figure 3.

The results of an exemplary experiment measuring the breakthrough of host cell protein in a solution mixture of influenza virus type A and DNA, passing through 0.8 ml columns containing S4FF (Control), QS4FF (90 $\mu$m) or QSBB (200 $\mu$m) beads is shown in Figure 4.

Table IX summarizes the results of the experiment described Figures 3 and 4, measuring the breakthrough titers of influenza virus Type A, HCP and host cell DNA in a solution mixture of the above, passed through 0.8 ml columns of either S4FF (Control), QS4FF (90 $\mu$m) or QSBB (200 $\mu$m) beads, in terms of recovery of influenza virus type A and removal of host cell protein after 42 mls of feed was passed through the columns.

**Table IX:**

| Sr. No. | Type of Bead | % Virus recovery | % HCP removal | Purity (ng/HAU) |
|---|---|---|---|---|
| 1. | S4FF (90 $\mu$m) | 100 | 0 | NA |
| 2. | QSFF(90 $\mu$m | 23 | 67 | 29 |
| 3. | QSBB(200 $\mu$m) | 68 | 62 | 11 |
| % Improved Recovery | | 66 | | |
| % Improved Purity | | | | 62 |

As summarized in Figures 3 and 4 and Table IX, it is evident that the larger 200 $\mu$m bead column has a much earlier break through of influenza virus type A and yields ~65% greater recovery of the virus than the 90$\mu$m beads. The host cell protein (HCP) break through curves of the QS4FF and QSBB columns look similar and the total HCP removal by the QSBB column appears to be little lower than the QS4FF column. But since the 200 $\mu$m beads have much higher recovery of the virus, the purity of the product obtained from this column is ~60% greater than the 90 $\mu$m bead column.

**Example 7: Comparison of the recovery of Influenza virus type A virus and removal of host cell protein from a mixture of influenza virus and host cell protein and DNA using mixtures of beads.**

In an exemplary experiment, flow-through purification of influenza virus type A from a mixture containing the virus and host cell proteins and host cell DNA using: (1) an anion exchange bead column; (2) a column containing anion and cation exchange beads in series; (3) a column containing anion exchange and hydrophobic beads in series; (4) a column containing anion exchange, cation exchange and hydrophobic beads in series; (5) or a column containing a mixture of anion exchange, cation exchange and hydrophobic beads.

Glass columns (Omnifit from Fisher Scientific Corporation) were packed with about 0.8 ml of positively charged Q Sepharose 4FF beads having an average diameter of 90 $\mu$m (QS4FF), or with 0.8 ml of positively charged Q Sepharose big beads having an average diameter of 200 $\mu$m (QSBB), or with 0.8 ml of Q Sepharose big beads followed by 0.8 ml of SP Sepharose big beads having an average diameter of 200 $\mu$m in series (AC), or with 0.8 ml of Q Sepharose big beads followed by 0.8 ml of Phenyl Sepharose big beads having an average diameter of 200 $\mu$m in series (AH), or with 0.8 ml of Q Sepharose big beads followed by 0.8 ml of SP Sepharose big beads followed by 0.8 ml of Phenyl Sepharose big beads having an average diameter of 200 $\mu$m in series (ACH), or with a mixture of Q Sepharose big beads, SP Sepharose big beads and Phenyl Sepharose big beads having an average diameter of 200 $\mu$m, to form a 2.4 ml column (MACH). The quality of the columns was verified using acetone pulse method to ensure they had good asymmetry and HETP and then equilibrated in 50 mM Trizma buffer at pH 8. (see, e.g., as described in PCT publication no. WO2008113011 A2, incorporated by reference herein)

Feed containing influenza virus type A was generated in a manner similar to that described in Example 6 and stored at -80°C until further use. Prior to conducting the experiment, the feed was thawed at 4°C overnight and then buffer exchanged into 50 mM Trizma buffer at pH 8.0 using a 10 Kilo-Dalton Centricon centrifugal filter device (Millipore Corporation). Approximately 60 ml of the buffer exchanged feed was passed through each column at a flow rate of 0.16 ml/min. The flow through the columns was collected in 1 ml fractions and assayed in duplicate for flu, host cell proteins, and host cell DNA content using HA assay, BCA assay and Pico-green assay respectively.

The results of an exemplary experiment measuring the breakthrough of influenza virus type A in a solution mixture of HCP and host cell DNA, passing through columns containing one of: 0.8 ml of QS4FF (90 $\mu$m) beads; 0.8 ml of QSBB (200 $\mu$m) beads; 0.8 ml of QSBB beads and 0.8 ml of SP Sepharose big beads (200 $\mu$m) in series (AC); 0.8 ml of QSBB beads and 0.8 ml of Phenyl Sepharose big beads (200 $\mu$m) in series (AH); 0.8ml of QSBB beads, 0.8 ml of SP Sepharose big beads and 0.8 ml of Phenyl Sepharose big beads in series (ACH); or 2.4 ml of a mixture of QSBB beads, SP Sepharose big beads and Phenyl Sepharose big beads (MACH) is shown in Figure 5.

The results of an exemplary experiment measuring the breakthrough of HCP in a solution mixture of influenza virus type A and host cell DNA, passing through columns containing one of: 0.8 ml of QS4FF (90 $\mu$m) beads; 0.8 ml of QSBB (200 $\mu$m) beads; 0.8 ml of QSBB beads and 0.8 ml of SP Sepharose big beads (200 $\mu$m) in series (AC); 0.8 ml of QSBB beads and 0.8 ml of Phenyl Sepharose big beads (200 $\mu$m) in series (AH); 0.8ml of QSBB beads, 0.8 ml of SP Sepharose big beads and 0.8 ml of Phenyl Sepharose big beads in series (ACH); or 2.4 ml of a mixture of QSBB beads, SP Sepharose big beads and Phenyl Sepharose big beads (MACH) is shown in Figure 6.

Table X summarizes the results of the experiment depicted in Figure 5 & 6, measuring the breakthrough titers of influenza virus Type A, HCP and host cell DNA in a solution mixture, after 42 mls of feed was passed through the various columns discussed herein.

**Table X**

| Type of Bead | % Virus recovery | % HCP removal | Purity (ng/HAU) | % Improved Recovery | % Improved Purity |
|---|---|---|---|---|---|
| QSFF (90 $\mu$m) | 57 | 82 | 22 | | |
| QSBB (200 $\mu$m) | 80 | 72 | 24 | 29 | |
| AC (200 $\mu$m) | 72 | 81 | 18 | 21 | 15 |
| AH (200 $\mu$m) | 72 | 84 | 15 | 21 | 29 |
| ACH (200 $\mu$m) | 71 | 86 | 14 | 20 | 36 |
| MACH (200 $\mu$m) | 67 | 88 | 12 | 15 | 44 |

The above calculations are assuming a theoretical buffer wash after the flow through process and assuming 40% void volume in the columns.

The above experiment describes the purification of influenza virus type A using single or multiple type of beads in a column. It can be seen from Figures 5 and 6 and Table X that using multiple types of beads slightly decreased the virus recovery. However, even with the larger volume, the columns with 200 $\mu$m beads always had higher virus recovery relative to the 90 $\mu$m beads due to their lower external surface area per unit volume and their limited interactions with the virus. Use of multiple type of beads, considerably improved the total HCP removal and purity of the product. This suggests that the external surface area added to the column due to the cation and hydrophobic beads did not interfere with the virus recovery, but the internal surface area of these beads allowed clearance of host cell proteins, thereby increasing the purity of the product. The study with multiple types of beads also suggests that similar purification could potentially be achieved using 200 $\mu$m mixed mode beads which combine the interactions of anion, cation and/or hydrophobic resins.

The specification is most thoroughly understood in light of the teachings of the references cited within the specification which are hereby incorporated by reference. The embodiments within the specification provide an illustration of embodiments in this invention and should not be construed to limit its scope. The skilled artisan readily recognizes that many other embodiments are encompassed by this invention. All publications and inventions are incorporated by reference in their entirety. To the extent that the material incorporated by reference contradicts or is inconsistent with the present specification, the present specification will supercede any such material. The citation of any references herein is not an admission that such references are prior art to the present invention.

Unless otherwise indicated, all numbers expressing quantities of ingredients, cell culture, treatment conditions, and so forth used in the specification, including claims, are to be understood as being modified in all instances by the term "about." Accordingly, unless otherwise indicated to the contrary, the numerical parameters are approximations and may vary depending upon the desired properties sought to be obtained by the present invention. Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the following claims.

Many modifications and variations of this invention can be made without departing from its spirit and scope, as will be apparent to those skilled in the art. The specific embodiments described herein are offered by way of example only and are not meant to be limiting in any way. It is intended that the specification and examples be considered as exemplary only, with a true scope and spirit of the invention being indicated by the following claims.

## Claims

1. A flow-through process for improving recovery of a large biomolecule from a sample comprising the large biomolecule and one or more contaminants, the process comprising contacting the sample with one or more populations of solid porous particles having a molecular weight cut off smaller than the large biomolecule being recovered, wherein at least one population of solid porous particle used in the flow-through process comprises a minimized external surface area per unit volume of the population of solid porous particle and an internal surface area which is not decreased by more than 25% per unit volume of population of solid porous particle, relative to a population of the solid porous particle which does not have a minimized external surface area per unit volume, thereby improving recovery of the large biomolecule.

2. The flow-through process of claim 1, wherein:

   (i) the one or more populations of solid porous particles are packed in a chromatography column, and/or
   (ii) the one or more populations of solid porous particles are selected from one or more of beads used for anion exchange chromatography, cation exchange chromatography and hydrophobic interaction chromatography, and/or
   (iii). at least one population of the solid porous particle has an average diameter ranging from 200 $\mu$m through 600 $\mu$m.

3. The flow-through process of any preceding claim, wherein the recovery of the large biomolecule is improved by at least 10%, or at least 20%, or at least 30%, or at least 40%, or at least 50%, or at least 60%, or at least 70%, or at least 80%, or at least 90%, over recovery of the large biomolecule using a flow-through process in which at least the one population of solid porous particles comprising a minimized external surface area per unit volume of the population of the particles and an internal surface area which is not decreased per unit volume of the population of the particles, is not used.

4. The flow-through process of claim 3, wherein the improved recovery comprises:

(i) an improved purity of the large biomolecule by at least 10%, and/or
(ii) a decrease in one or more contaminants by at least 10%, or at least 20%, or least 30%, or at least 40% or more.

5. The flow-through process of any preceding claim, wherein the one or more populations of solid porous particles comprises mixed mode chromatography beads.

6. The flow-through process of claim 5, wherein the encapsulated virus is:

(i) an influenza virus, or
(ii) an adenovirus, or
(iii) bacteriophage.

7. The flow-through process of claim 5, wherein the virus-like particle is a styrene particle coated with BSA.

8. The flow-through process of claim 7, wherein the influenza virus is an H1N1 virus.

9. The flow-through process of any preceding claim, wherein the large biomolecule is selected from the group consisting of an encapsulated virus, a virus-like particle and a conjugate vaccine.

10. The flow-through process of claim 9, wherein the proteins are host cell proteins.

11. The flow-through process of any preceding claim, wherein the one or more contaminants is selected from the group consisting of nucleic acid, proteins, excipients and cell culture additives.

12. The flow-through process of any preceding claim, wherein the large biomolecule is produced in a cell culture or in eggs.

13. The flow-through process of claim 8, wherein the cell culture comprises MDCKcells.

14. The flow-through process of any preceding claim, wherein the sample is a cell culture supernatant.

15. The flow-through process of any preceding claim, wherein the process enables a recovery of at least 50%, or at least 55%, or at least 60%, or at least 65%, or at least 70%, or at least 75%, or at least 80%, or at least 85%, or at least 90% of the large biomolecule in the sample.

*Fig.1*

*Fig.2*

Fig.3

Fig.4

Fig.5

Fig.6

Europäisches
Patentamt

European
Patent Office

Office européen
des brevets

# EUROPEAN SEARCH REPORT

**Application Number**

EP 10 19 4894

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/131526 A1 (GE HEALTHCARE BIO SCIENCES AB [SE]; BERGSTROEM JAN [SE]; GLAD GUNNAR [])<br>29 October 2009 (2009-10-29)<br>* abstract *<br>* page 6, line 5 *<br>* page 6, line 20 - line 22 *<br>* page 8, lines 25-28 *<br>* page 15, line 5 *<br>* example 3 *<br>* tables 4,5 *<br>----- | 1-4,6-15 | INV.<br>C12N7/02<br>B01D15/08 |
| X | WO 2004/101765 A2 (GIBSON MARYLOU [US]; ROBBINS JOAN [US])<br>25 November 2004 (2004-11-25)<br>* abstract *<br>* claims 3,5 *<br>* page 3, line 30 - line 31 *<br>* page 6, line 1 - line 2 *<br>* example 1 *<br>----- | 1-4,<br>9-12,14,<br>15 | |
| X | WO 2009/020609 A2 (NANOGEN INC [US]; WEISBURG WILLIAM G [US]; MATHER ELIZABETH L [US]; HA) 12 February 2009 (2009-02-12)<br>* abstract *<br>* page 2, line 26 ff. *<br>* page 3, line 11 ff. *<br>* page 12, line 13 - line 24 *<br>* page 14, line 22 ff. *<br>* page 20, line 24 ff. *<br>-----<br><br>-/-- | 1-5,<br>9-12,14,<br>15 | **TECHNICAL FIELDS SEARCHED (IPC)**<br>C12N<br>B01D |

The present search report has been drawn up for all claims

| Place of search | Date of completion of the search | Examiner |
|---|---|---|
| Munich | 21 April 2011 | Brero, Alessandro |

EPO FORM 1503 03.82 (P04C01)

EP 2 336 304 A1

Europäisches
Patentamt

European
Patent Office

Office européen
des brevets

**EUROPEAN SEARCH REPORT**

Application Number

EP 10 19 4894

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LU ET AL: "Polyethylene glycol increases purification and recovery, alters retention behavior in flow-through chromatography of hemoglobin", JOURNAL OF CHROMATOGRAPHY, ELSEVIER SCIENCE PUBLISHERS B.V, NL, vol. 1059, no. 1-2, 3 December 2004 (2004-12-03), pages 233-237, XP005003932, ISSN: 0021-9673, DOI: DOI:10.1016/J.CHROMA.2004.10.047 * abstract * * paragraph [02.2] * | 1-4,11, 12,15 | |
| X | MEGHROUS J ET AL: "Development of a simple and high-yielding fed-batch process for the production of influenza vaccines", VACCINE, ELSEVIER LTD, GB, vol. 28, no. 2, 11 December 2009 (2009-12-11), pages 309-316, XP026791365, ISSN: 0264-410X [retrieved on 2009-10-30] * abstract * * paragraph [02.8] * | 1-4,11, 12,15 | |
| A | NOAD R ET AL: "Virus-like particles as immunogens", TRENDS IN MICROBIOLOGY, ELSEVIER SCIENCE LTD., KIDLINGTON, GB, vol. 11, no. 9, 1 September 2003 (2003-09-01), pages 438-444, XP003004438, ISSN: 0966-842X, DOI: DOI:10.1016/S0966-842X(03)00208-7 * abstract * | 7 | TECHNICAL FIELDS SEARCHED (IPC) |

-/--

The present search report has been drawn up for all claims

| Place of search | Date of completion of the search | Examiner |
|---|---|---|
| Munich | 21 April 2011 | Brero, Alessandro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

23

Europäisches
Patentamt
European
Patent Office
Office européen
des brevets

# EUROPEAN SEARCH REPORT

Application Number

EP 10 19 4894

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JUNGBAUER A: "Chromatographic media for bioseparation", JOURNAL OF CHROMATOGRAPHY, ELSEVIER SCIENCE PUBLISHERS B.V, NL, vol. 1065, no. 1, 11 February 2005 (2005-02-11), pages 3-12, XP004750085, ISSN: 0021-9673, DOI: DOI:10.1016/J.CHROMA.2004.08.162 * the whole document * ----- | 1-15 | |
| | | | TECHNICAL FIELDS SEARCHED (IPC) |

The present search report has been drawn up for all claims

| Place of search | Date of completion of the search | Examiner |
|---|---|---|
| Munich | 21 April 2011 | Brero, Alessandro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                  
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 19 4894

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2011

| Patent document cited in search report | | Publication date | Patent family member(s) | | Publication date |
|---|---|---|---|---|---|
| WO 2009131526 | A1 | 29-10-2009 | AU 2009238686 A1<br>CN 102015094 A<br>EP 2268393 A1<br>US 2011045574 A1 | | 29-10-2009<br>13-04-2011<br>05-01-2011<br>24-02-2011 |
| WO 2004101765 | A2 | 25-11-2004 | US 2005009168 A1 | | 13-01-2005 |
| WO 2009020609 | A2 | 12-02-2009 | US 2009048439 A1 | | 19-02-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61284368 A
- WO 2008073490 A
- WO 2004112707 A
- EP 1801697 A1
- US 20050118140 A
- WO 2008113011 A2

### Non-patent literature cited in the description

- **TRANSFIGURACUIN et al.** *J Virol Methods,* 2007, vol. 142, 21-28
- **KALBFUSS et al.** *Biotech. Bioeng.,* 2007, vol. 96, 932-944
- **WOLFGANG HAISS et al.** *etermination of Size and Concentration of Gold Nanoparticles from UV-Vis Spectra,* 2007, vol. 79, 4215-4221
- **S. BRUNAUER et al.** *J. Am. Chem. Soc.,* 1938, vol. 60, 309
- **OPITZ et al.** *Biotech. Bioengg.,* 2009, vol. 103, 1144-1154
- **OPITZ et al.** *J. Biotech.,* 2007, vol. 131, 309-317
- **NAYAK et al.** *J. Chrom.,* 2005, vol. 823, 75-81
- **DULBECCO et al.** Symp. Quant. Biol. Cold Spring Harbor, 1953, vol. 18, 273-279
- Methods in Molecular Biology. **SPACKMAN, E.** Avian Influenza Virus. Humana Press, 436
- **NERMUT et al.** *AIDS Res.,* 1993, vol. 9, 929-938
- **GU et al.** *Enzy. Micro. Tech.,* 2003, vol. 33, 430-437
- **DEPHILLIPS.** *J. Chrom. A.,* 2003, vol. 883, 39-54
- **SOBISCH et al.** *Colloid Poly. Sci.,* 2006, vol. 133, 169-172
- **BRUNAUER et al.** *J. Am. Chem. Soc.,* 1938, vol. 60, 309-319
- **WULFF et al.** *Therchimi. Acta,* 2004, vol. 419, 291-294
- Ion Exchange Chromatography and Chromatofocusing- Principles and Methods. Amersham Biosciences